# EUROPEAN PATENT APPLICATION

(11) **EP 1 935 373 A2**
(43) Date of publication of application: **25.06.2008**
(21) Application number: 07024518.8
(22) Date of filing: 18.12.2007
(51) Int. Cl.: A61F 2/06, A61B 19/00

(54) **Prosthesis deployment apparatus comprising an electromagnetic marker**

(30) Priority: 18.12.2006 US 612123
(71) Applicant: Medtronic Vascular, Inc., Santa Rosa, CA 95403 (US)
(72) Inventor: Mciff, Greg, Santa Rosa CA 95409 (US); Yamasaki, Dwayne S., Rohnert Park CA 94928 (US); Simon, David, Boulder Colorado 80303 (US); Gardeski, Kenneth, Plymouth Minnesota 55442 (US)
(74) Representative: Zimmermann, Gerd Heinrich

(57) **Abstract**

A marker implant apparatus (100) includes an electromagnetic marker (102) adapted for implantation in a human patient; and a fastener (104) secured to said electromagnetic marker and adapted to engage tissue, said fastener (104) having a helical configuration and a piercing end (104a) configured to pierce tissue. Furthermore, a bifurcated tubular prosthesis is provided which includes a bifurcated tubular member having a proximal end and first and second tubular leg portions that extend in a direction distal to said proximal end, each tubular leg portion having an open end; and an electromagnetic marker secured to one of the tubular leg portions in the vicinity of the open end of that portion.

## Description

### FIELD OF THE INVENTION

The invention relates to prosthesis deployment apparatus for prosthesis deployment in the human vascular system and/or post deployment management.

### BACKGROUND OF THE INVENTION

Tubular prostheses such as stents, grafts, and stent-grafts (e.g., stents having an inner and/or outer covering comprising graft material and which may be referred to as covered stents) have been widely used in treating abnormalities in passageways in the human body. In vascular applications, these devices often are used to replace or bypass occluded, diseased or damaged blood vessels such as stenotic or aneurysmal vessels. For example, it is well known to use stent-grafts, which comprise biocompatible graft material (e.g., Dacron ® or expanded polytetrafluoroethylene (ePTFE)) supported by a framework (e.g., one or more stent or stent-like structures), to treat or isolate aneurysms. The framework provides mechanical support and the graft material or liner provides a blood barrier. The graft material for any of the prostheses described herein also can be any suitable material such as Dacron ® material or expanded polytetrafluoroethylene (ePTFE).

Aneurysms generally involve abnormal widening of a duct or canal such as a blood vessel and generally appear in the form of a sac formed by the abnormal dilation of the duct or vessel. The abnormally dilated vessel has a wall that typically is weakened and susceptible to rupture. Aneurysms can occur in blood vessels such as in the abdominal aorta where the aneurysm generally extends below the renal arteries distally to or toward the iliac arteries.

In treating an aneurysm with a stent-graft, the stent-graft typically is placed so that one end of the stent-graft is situated proximally or upstream of the diseased portion of the vessel and the other end of the stent-graft is situated distally or downstream of the diseased portion of the vessel. In this manner, the stent-graft spans across and extends through the aneurysmal sac and beyond the proximal and distal ends thereof to replace or bypass the weakened portion. The graft material typically forms a blood impervious lumen to facilitate endovascular exclusion of the aneurysm.

Such prostheses can be implanted in an open surgical procedure or with a minimally invasive endovascular approach. Minimally invasive endovascular stent-graft use is preferred by many physicians over traditional open surgery techniques where the diseased vessel is surgically opened, and a graft is sutured into position bypassing the aneurysm. The endovascular approach, which has been used to deliver stents, grafts, and stent grafts, generally involves cutting through the skin to access a lumen of the vasculature. Alternatively, lumenar or vascular access may be achieved percutaneously via successive dilation at a less traumatic entry point. Once access is achieved, the stent-graft can be routed through the vasculature to the target site. For example, a stent-graft delivery catheter loaded with a stent-graft can be percutaneously introduced into the vasculature (e.g., into a femoral artery) and the stent-graft delivered endovascularly to a portion where it spans across the aneurysm where it is deployed.

When using a balloon expandable stent-graft, balloon catheters generally are used to expand the stent-graft after it is positioned at the target site. When, however, a self-expanding stent-graft is used, the stent-graft generally is radially compressed or folded and placed at the distal end of a sheath or delivery catheter and self expands upon retraction or removal of the sheath at the target site. More specifically, a delivery catheter having coaxial inner and outer tubes arranged for relative axial movement therebetween can be used and loaded with a compressed self-expanding stent-graft. The stent-graft is positioned within the distal end of the outer tube (sheath) and in front of a stop fixed to the distal end of the inner tube. Regarding proximal and distal positions referenced herein, the proximal end of a prosthesis (e.g., stent-graft) is the end closest to the heart (by way of blood flow) whereas the distal end is the end furthest away from the heart during deployment. In contrast, the distal end of a catheter is usually identified as the end that is farthest from the operator, while the proximal end of the catheter is the end nearest the operator. Once the catheter is positioned for deployment of the stent-graft at the target site, the inner tube is held stationary and the outer tube (sheath) is withdrawn so that the stent-graft is gradually exposed and expands. An exemplary stent-graft delivery system is described in U.S. Patent Application Publication No. 2004/0093063, which published on May 13, 2004 to Wright et al. and is entitled Controlled Deployment Delivery System, the disclosure of which is hereby incorporated herein in its entirety by reference.

Although the endovascular approach is much less invasive, and usually requires less recovery time and involves less risk of complication as compared to open surgery, there can be concerns with alignment of asymmetric features of various prostheses in relatively complex applications such as one involving branch vessels. Branch vessel techniques have involved the delivery of a main device (e.g., a graft or stent-graft) and then a secondary device (e.g., a branch graft or branch stent-graft) through a fenestration or side opening in the main device and into a branch vessel.

The procedure becomes more complicated when more than one branch vessel is treated. One example is when an aortic abdominal aneurysm is to be treated and its proximal neck is diseased or damaged to the extent that it cannot support a reliable connection with a prosthesis. In this case, grafts or stent-grafts have been provided with fenestrations or openings formed in their side wall below a proximal portion thereof. The fenestrations or openings are to be aligned with the renal arteries and the proximal portion is secured to the aortic wall above the renal arteries.

To ensure alignment of the prostheses fenestrations and branch vessels, current techniques involve placing guidewires through each fenestration and branch vessel (e.g., artery) prior to releasing the main device or prosthesis. This involves manipulation of multiple wires in the aorta at the same time, while the delivery system and stent-graft are still in the aorta. In addition, an angiographic catheter, which may have been used to provide detection of the branch vessels and preliminary prosthesis positioning, may still be in the aorta. Not only is there risk of entanglement of these components, the openings in an off the shelf prosthesis with preformed fenestrations may not properly align with the branch vessels due to differences in anatomy from one patient to another. Prostheses having preformed custom located fenestrations or openings based on a patient's CAT scans also are not free from risk. A custom designed prosthesis is constructed based on a surgeon's interpretation of the scan and still may not result in the desired anatomical fit. Further, relatively stiff catheters are used to deliver grafts and stent-grafts and these catheters can apply force to tortuous vessel walls to reshape the vessel (e.g., artery) in which they are introduced. When the vessel is reshaped, even a custom designed prosthesis may not properly align with the branch vessels.

Generally speaking, physicians often use fluoroscopic imaging techniques to confirm that a catheter and or stent-graft is properly positioned during deployment. Fluoroscopy allows one to observe real-time X-ray images of the aorta and the area of interest and has been used to identify branch vessels such as the renal arteries for assisting in prosthesis positioning. However, this approach requires one to administer a radiopaque substance, which generally is referred to as a contrast medium, agent or dye, into the patient so that it reaches the area to be visualized (e.g., the renal arteries). A catheter can be introduced through the femoral artery in the groin of the patient and endovascularly advanced to the vicinity of the renals. The fluoroscopic images of the transient contrast agent in the blood, which can be still images or real-time motion images, allow two dimensional visualization of the location of the renals.

The use of X-rays, however, requires that the potential risks from a procedure be carefully balanced with the benefits of the procedure to the patient. While physicians always try to use low dose rates during fluoroscopy, the length of a procedure may be such that it results in a relatively high absorbed dose to the patient. Patients who cannot tolerate contrast enhanced imaging or physicians who must reduce radiation exposure need an alternative approach for monitoring stent-graft positioning and deployment.

Another challenge with fluoroscopy is that to enable navigation of a catheter to a branch artery such as a renal artery requires registration of where the device is in relation to vascular anatomy. This is difficult due to the natural movement of vascular anatomy with respiration and cardiac cycles and the change that is imposed on the vessel when catheters and wires are introduced.

U.S. Patent No. 5,617,878 to Taheri discloses a method comprising interposition of a graft at or around the intersection of major arteries and thereafter, use of intravenous ultrasound or angiogram to visualize and measure the point on the graft where the arterial intersection occurs. A laser or cautery device is then interposed within the graft and used to create an opening in the graft wall at the point of the intersection. A stent is then interposed within the graft and through the created opening of the intersecting artery.

U.S. Patent Application Serial No. 11/276,512 to Marilla, entitled Multiple Branch Tubular Prosthesis and Methods, filed March 3, 2006, and co-owned by the assignee of the present application discloses positioning in an endovascular prosthesis an imaging catheter (intravenous ultrasound device (IVUS)) having a device to form an opening in the side wall of the prosthesis. The imaging catheter detects an area of the prosthesis that is adjacent to a branch passageway (e.g., a renal artery), which branches from the main passageway in which the prosthesis has been deployed. The imaging catheter opening forming device is manipulated or advanced to form an opening in that area of the prosthesis to provide access to the branch passageway. The imaging catheter also can include a guidewire that can be advanced through the opening.

There remains a need for alternative prosthesis delivery and post operative monitoring methods and apparatus.

### SUMMARY OF THE INVENTION

The present invention involves improvements in prosthesis deployment apparatus.

In one embodiment of the invention, a marker implant apparatus comprises an electromagnetic marker adapted for implantation in a human patient; and a fastener secured to the electromagnetic marker and adapted to engage tissue, the fastener having a helical configuration and a piercing end configured to pierce tissue.

According to another embodiment of the invention, a bifurcated tubular prosthesis comprises a bifurcated tubular member having a proximal end and first and second tubular leg portions that extend in a direction distal to the proximal end, each tubular leg portion having an open end; an electromagnetic marker secured to one of the tubular leg portions in the vicinity of the open end of that portion.

According to another embodiment of the invention, a prosthesis delivery apparatus comprises a guidewire adapted for endovascular delivery in a patient; and a leadless electromagnetic marker secured to the guidewire.

Other features, advantages, and embodiments according to the invention will be apparent to those skilled in the art from the following description and accompanying drawings.

### BRIEF DESCRIPTION OF THE DRAWINGS

FIG. 1 diagrammatically illustrates one embodiment of a position locator marker delivery system in accordance with the invention.

FIG. 1A is diagrammatically illustrates implantation of a plurality of position locator markers implanted about the ostia of a branch vessel in accordance with the invention.

FIG. 2A illustrates a marker implant device embodiment according to the invention;

FIG. 2B illustrates the marker implant device of FIG. 2A implanted in a vessel wall.

FIG. 3A illustrates another marker implant device embodiment according to the invention;

FIG. 3B illustrates the marker implant apparatus of FIG. 3A implanted in a vessel wall.

FIG. 4A illustrates delivery apparatus loaded with the marker device shown in FIG. 3.

FIG. 4B illustrates the delivery apparatus of FIG. 4A deploying the marker device of FIG. 3.

FIG. 5A illustrates one prosthesis delivery system for use in one method embodiment of the invention.

FIG. 5B illustrates the prosthesis delivery system of FIG. 5A in a partially deployed state.

FIG. 5C illustrates the prosthesis delivery system of FIG. 5B with the prosthesis proximal end deployed.

FIGS. 6A-C diagrammatically illustrate one method of the invention where FIG. 6A illustrates prosthesis delivery to a desired site; FIG. 6B illustrates partial deployment of the prosthesis; and FIG. 6C illustrates the prosthesis deployed.

FIGS. 7A-B diagrammatically illustrate delivery and deployment of a contralateral leg according to another embodiment of the invention where FIG. 7A illustrates using a marker to assist with cannulation of the contralateral stump of the prosthesis of FIG. 6C and FIG. 7B illustrates the contralateral leg deployed and secured to the stump after cannulation.

FIG. 8 diagrammatically illustrates prosthesis position after deployment.

FIG. 9 diagrammatically illustrates post operative prosthesis position, which is compared with the data of FIG. 8.

FIG. 10A diagrammatically illustrates a known system for energizing and locating leadless electromagnetic markers.

FIG. 10B is a schematic isometric view of the receiver of FIG. 10A.

FIG. 10C is a diagrammatical section view of a known leadless electromagnetic marker.

### DETAILED DESCRIPTION

The following description will be made with reference to the drawings where when referring to the various figures, it should be understood that like numerals or characters indicate like elements.

Regarding proximal and distal positions, the proximal end of the prosthesis (e.g., stent-graft) is the end closest to the heart (by way of blood flow) whereas the distal end is the end farthest away from the heart during deployment. In contrast, the distal end of the catheter is usually identified as the end that is farthest from the operator, while the proximal end of the catheter is the end nearest the operator. Therefore, the prosthesis (e.g., stent-graft) and delivery system proximal and distal descriptions may be consistent or opposite to one another depending on prosthesis (e.g., stent-graft) location in relation to the catheter delivery path.

Furthermore, in the following reference will be made to electromagnetic markers. It should be understood by those skilled in the art that in the context of the present application the term "electromagnetic marker" is used for devices responsive to magnetic fields, especially pulsed magnetic fields. In particular, the term "electromagnetic marker" relates to devices adapted to absorb energy from a pulsed magnetic field by means of electromagnetic induction. Furthermore, it will be understood by those skilled in the art that the term "electromagnetic markers" as used in the present application does not relate to fluoroscopic markers although also X-rays and other forms of visible or invisible light radiation may be considered a specific form of electromagnetic radiation.

According to one embodiment of the invention, one or more localization markers are implanted at a target site in a patient's vessel to provide a virtual image of a portion of the vessel and/or provide a target for deploying a prosthesis. The target site can be at various locations along the vessel including locations along a branch vessel including, but not limited to, the region of the branch vessel ostia. Branch vessels can occur in or around the intersection of a vessel (e.g., the aorta) and other attendant vessels (e.g., major arteries such as the renal, brachiocephalic, subclavian and carotid arteries). In one embodiment, a leadless electromagnetic marker and system for generating a magnetic field for excitation of the leadless marker is used.

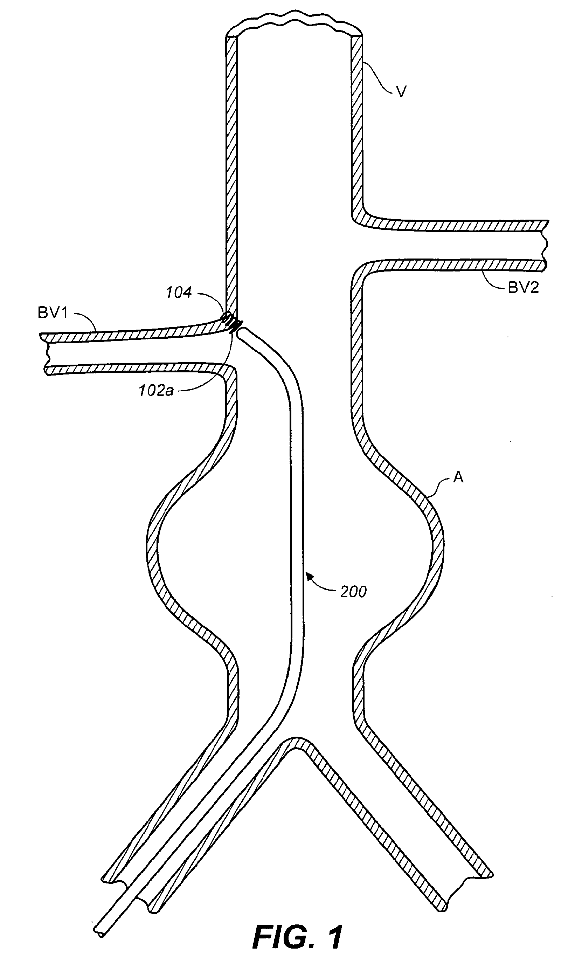

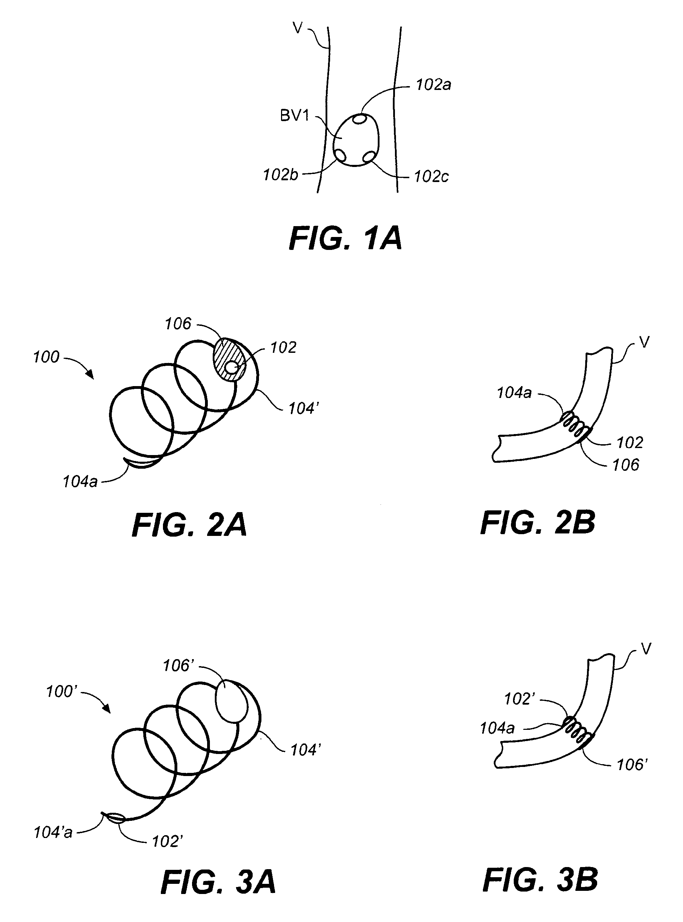

Referring to FIG. 1, a method of implanting a localization marker in a vessel according to one embodiment of the invention is shown. In the illustrative embodiment, steerable marker delivery catheter 200, which has a remotely actuable deflectable distal tip (steerable catheters with remotely actuable deflectable distal tips are well known) is advanced through a vessel V, beyond aneurysm A to branch vessel BV1, which is below branch vessel BV2. Vessel V can be the aorta and branch vessels BV1 and BV2 can be the renal arteries. In this example, the distal end portion of marker delivery catheter 200 is remotely actuated to bend and steer the catheter tip toward the branch vessel ostia and to contact a portion of the branch vessel in the vicinity of the ostia with the catheter tip. Conventional fluoroscopic techniques can be used to position the catheter tip at the desired location. A leadless localization marker 102a is then delivered and secured to the vessel with fastener 104, which will be described in more detail below.

Although one localization marker is shown, a plurality of localization markers can be used. The particular number of markers typically depends on the span of markers desired to be imaged or the area desired to be virtually represented. In the example shown in FIG. 1A, three lead less markers 102a, 102b, and 102c are shown placed along the ostia of branch vessel BV1. In this manner, the markers can assist in creating a real-time virtual image of the ostia or directly provide a real-time positional guide since they can be represented on a display monitor to guide a prosthesis (e.g., such as a stent-graft) to a target site above or below the branch vessel. The markers can be equidistantly spaced from one another as shown or spaced otherwise depending on the desired nature of the virtual image thereof or virtual target created thereby. They also can be placed in different configurations. For example, they can be placed along the bottom portion of the ostia or below the ostia in vessel V to provide a reference for positioning the prosthesis in the vessel.

Referring to FIGS. 2A and 2B, one embodiment of a leadless localization marker with an anchor mechanism for fastening the marker to a vessel is shown and generally designated with reference numeral 100. Apparatus 100 includes helical fastener 104, which has a pointed lead end 104a that is adapted to penetrate tissue, and a trailing end that has a cross member 106 crossing the diameter of the trailing coil of the fastener and forming a stop to prevent or limit further penetration of fastener 104 in the vessel. Helical member 104 also can be provided with barbs (not shown) to assist in anchoring helical member 104 in the vessel wall. In this embodiment, cross member 106 provides a platform to which marker 102 (which can correspond to localization any of markers 102a, 102b, 102c...102n) is secured. The localization marker can be secured to the platform with using any suitable means such as biocompatible glue (e.g., cyanoacylate). FIG. 2B illustrates the apparatus secured to a vessel after helical fastener 104 has been sufficiently turned so that cross member 106 contacts an inner portion of the vessel wall to secure marker 102 thereto.

Referring to FIGS. 3A and 3B, another embodiment of a localization marker with an anchor mechanism is shown and generally designated with reference numeral 100'. Apparatus 100' includes helical fastener 104', which has a pointed lead end 104'a and cross member 106' crossing the diameter of the trailing coil of the fastener and forming a stop to prevent or limit further penetration of fastener 104' in the vessel wall. Elements 100', 104', 104'a and 106' can be the same as elements 100, 104, 104a, and 106. Although marker 102' can have the same construction as marker 102, in this embodiment marker 102' is secured to the lead portion of fastener 104' with any suitable mean such as biocompatible adhesive (e.g., cyanoacylate). FIG. 3B illustrates apparatus 100' secured to a vessel after helical fastener 104 has been sufficiently turned to where stop 106' contacts the vessel inner wall. In this position, marker 102' is either external to the vessel (as shown) or in the vessel wall depending on the length of the helical fastener.

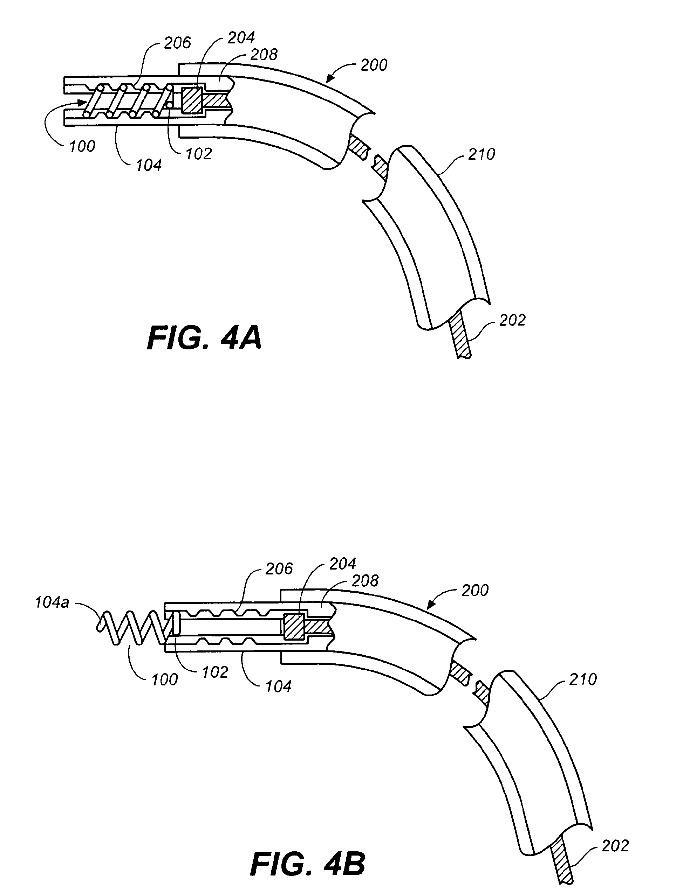

Referring to FIGS. 4A and 4B, marker apparatus 100 is shown loaded in a delivery system corresponding to that described in U.S. Patent No. 6,960,217 to Bolduc, entitled Endovascular Aneurysm Repair System, the disclosure of which is hereby incorporated herein in its entirety by reference thereto. FIG. 4A shows implant apparatus 100 in a state suitable for delivery to a desired site and FIG. 4B shows the implant apparatus being deployed. To deploy helical fastener 104, it is rotated via a fastener driver 204 through a drive shaft 202, which is secured to the fastener driver. The drive shaft proximal end can be directly rotated by hand or coupled to a control assembly such as the control assembly described in U.S. Patent No. 6,960,217 *(supra).* Drive shaft 202 can be made of any material that allows for both bending and torque transmission capability. The drive shaft is connected to fastener driver 204, which engages and imparts torque to the helical fastener as shown in FIG. 4B. Rotation of the fastener driver 204, causes rotation of a diagonal (cross) member 102 of the screw 104 through a pair of centrally located axially extending drive members, such as a set of horizontal pins or bars (not easily viewed in the figure) which are fixed to and can transmit torque to fastener driver 204 and turn the screw and allow it to moves in the groove 206 of the stationary surrounding of the fastener applier 208 which is fixed to the outer cover 210. Rotation of the fastener driver 204 causes rotation of the horizontal drive pins to turn the helical fastener 104a in the (thread) groove 206. The rotation of the helical fastener (screw) 204 moves it forward and out of the end of the fastener applier 208 and into the target tissue.

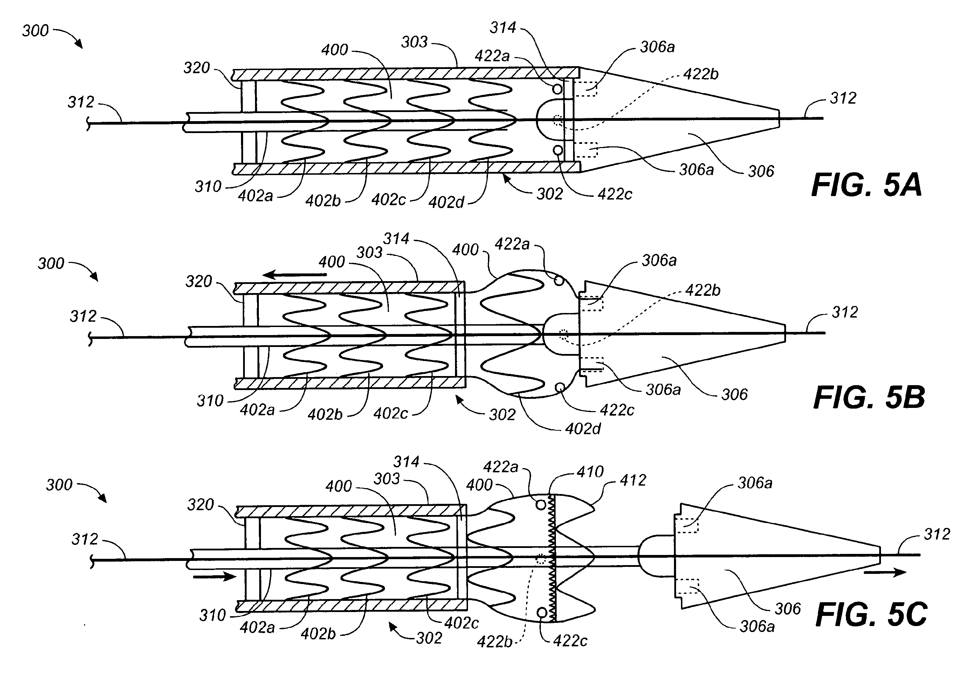

Referring to FIGS. 5A-C, one delivery catheter system configuration according to the invention is shown in a pre-deployment loaded state (FIG. 5A) and two partial deployment states (FIG. 5B & 5C) and generally designated with reference numeral 300. System 300 comprises delivery catheter 302, which includes catheter sheath 303, which can be referred to as an outer tube, inner guidewire tube 310 for tracking over guidewire 312, and flexible tapered tip (or tapered tip) 306. Sheath 303 and guidewire tube 310 are coaxial and arranged for relative axial movement therebetween. The prosthesis (e.g., stent-graft 400) is positioned within the distal end of outer tube 303 and in front of pusher member or stop 320, which is concentric with and secured to inner guidewire tube 310 and can have a disk or ring shaped configuration with a central access bore to provide access for guidewire tube 310. Although three markers 122a, 122b, and 122c are shown attached to the proximal end portion of the prosthesis, one or more markers can be used depending on the desired nature of the image to be created. The markers can be sutured to the prosthesis or attached thereto with other suitable means.

In the example where prosthesis 400 comprises a stent-graft as shown in the illustrative embodiment, the stent graft comprises a tubular graft member and a plurality of annular undulated stent elements, such as stent elements 402a,b,c,d, etc. to provide structural support to the graft as is known in the art. An undulating bear spring element 412 also can be sutured or otherwise attached to the proximal end of the prosthesis and/or an annular undulating wire 410 secured to the proximal end of the prosthesis to provide radial strength as well. Spring element 412 has a radially outward bias so that when it is released from a radially collapsed or restrained state it expands outwardly to secure the proximal portion of the prosthesis to the target passageway wall. Another undulating wire 410 can be attached to the prosthesis distal end as well or in the alternative. More specifically, a support spring 410 can be provided at one or both ends of the prosthesis. The stent and support elements can be positioned on the interior and/or exterior of the graft member and secured thereto by suturing or other conventional means.

A radiopaque ring 314 can be provided on the inside of the distal end portion of sheath 303 in overlapping relation to tapered tip 306 (FIG. 3A) to assist with imaging distal end of sheath 303 when using fluoroscopic techniques if desired. Alternatively, optional radiopaque ring 314 can be provided on the proximal end of the tapered tip. Once the catheter is positioned for deployment of the prosthesis at the desired site, the inner member or guidewire lumen 310 with stop 320 are held stationary and the outer tube or sheath 303 withdrawn so that tapered tip 306 is displaced from sheath 303 and the stent-graft gradually exposed and allowed to expand. Stop 320 therefore is sized to engage the distal end of the stent-graft as the stent-graft is deployed. The proximal ends of the sheath 303 and inner tube or guidewire lumen 112 are coupled to and manipulated by a conventional handle (not shown) external to the patient. Tapered tip 306 optionally can be configured with an annular recess or cavity 306a formed in its proximal end portion and configured to receive and retain the proximal end portion of the prosthesis (e.g., by holding the end of bare spring element 212) in a radially compressed configuration before allowing expansion thereof during a later phase of its deployment. Alternatively, any of the stent-graft deployment systems described in U.S. Patent Application Publication No. 2004/0093063, which published on May 13, 2004 to Wright et al. and is entitled Controlled Deployment Delivery System, the disclosure of which is hereby incorporated herein by reference in its entirety, can be incorporated into stent-graft delivery system 300.

Prosthesis deployment is illustrated in FIGS. 5B and 5C. In FIG. 5B, the prosthesis delivery system is shown with catheter sheath 303 partially pulled back and a portion of the prosthesis partially expanded. In this partially retracted position, the proximal end of the prosthesis is constrained allowing the prosthesis to be repositioned (e.g., longitudinally or rotationally moved) if desired before release of the proximal end of the prosthesis. The surgeon can determine if prosthesis repositioning is desired based on the relative positions of the implanted marker or markers and the marker or markers on the prosthesis, which can be determined with system 600 described below and displayed on a monitor (not shown). In FIG. 5C, the catheter sheath is held stationary and guidewire lumen 310, which is fixedly secured to tapered tip 306, is advanced to separate tapered tip 306 from catheter sheath 303 and release the proximal end of prosthesis 400.

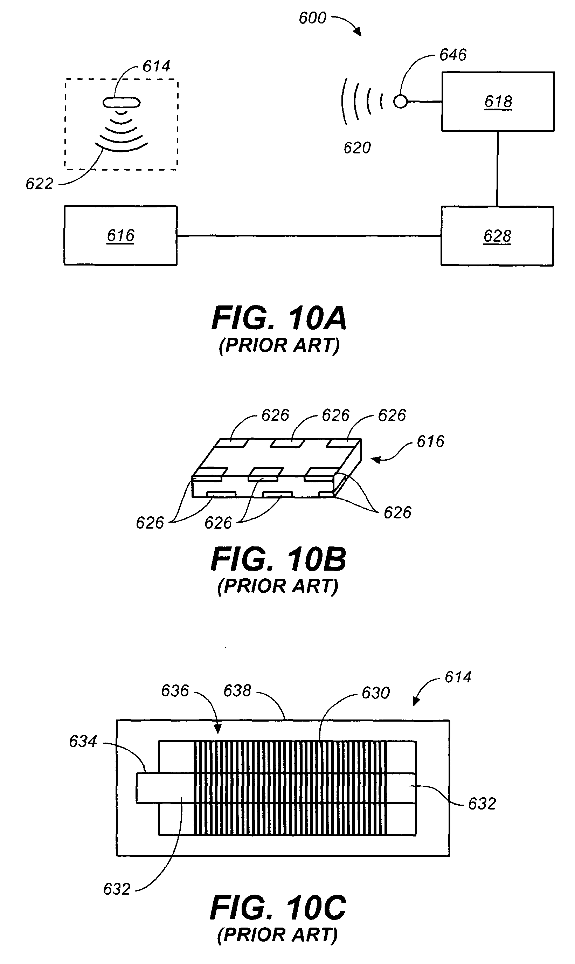

The following illustrates exemplary procedures incorporating embodiments of the invention. For the purposes of the example, the procedures involve the endovascular delivery and deployment of an AAA bifurcated stent-graft in a patient's aorta below the renal arteries, subsequent contralateral leg cannulation, and post-operative monitoring. In this example, system 600 for energizing and locating one or more leadless electromagnetic resonating markers and the lead less electromagnetic resonating markers shown in FIGS. 10-C and which will be described hereafter will be used. Further, all of the leadless markers described herein can have the same construction as that shown in FIG. 10C.

Prior to the surgical procedure, the patient is scanned using either a Contrast Enhanced CT or MRI scanner to generate a three-dimensional model of the vasculature to be tracked. Therefore, the aorta and branch vessels of interest (e.g., renal arteries) can be scanned and images taken thereof to create a three-dimensional pre-procedural data set for that vasculature and create a virtual model upon which real-time data will be overlaid. The virtual model is input into the software that consolidates all of the navigation information and displays information that the user sees. While creation and use of a virtual model is desirable for any or all of the following steps: marker implantation, stent-graft deployment, or cannulation, it can be performed without a virtual model.

The magnetic field generators of system 600 are positioned on the operating table or sufficiently close to the patient to facilitate triangulation of the exact position of sensors in three-dimensional space using xyz coordinates.

The patient is prepared for surgery and the femoral artery is exposed via a small surgical incision and access to the lumen is gained with an introducer sheath. A guidewire is inserted and followed under fluoroscopy to the area of interest.

Anatomical reference points of the patient in the magnetic field are or have been placed as a reference, either external to the patient or intravascularly. Examples of external markers are fiducial markers on the patient's skin over vertebra or on skin over the iliac crest. Examples of internal markers are markers placed in the wall of the renals arteries of other branches of the aorta. A contrast agent catheter is delivered through the femoral artery and the vasculature perfused with contrast and a fluoroscopic image up to the renal arteries taken. The processor orients or registers the three-dimensional virtual image to the fluoroscopic X-ray image. Such registration can be performed at the time of the surgery using an O-arm prior to the subsequent procedure taking place.

The processor orients or registers the three-dimensional virtual image to the fluoroscopic X-ray image. Registration occurs after the placement of the leadless markers by activating the navigation system and the electromagnetic field then obtaining a contrast enhanced subtraction run of the target area. The navigation software then registers the vascular anatomy to the pre-op 3D reconstruction. Such registration is known in the art.

Leadless markers 102a, 102b, and 102c are delivered to the lower renal artery ostia as shown in FIG. 1 using fluoroscopic techniques and secured to the renal artery along the ostia thereof as shown in FIG. 1A.

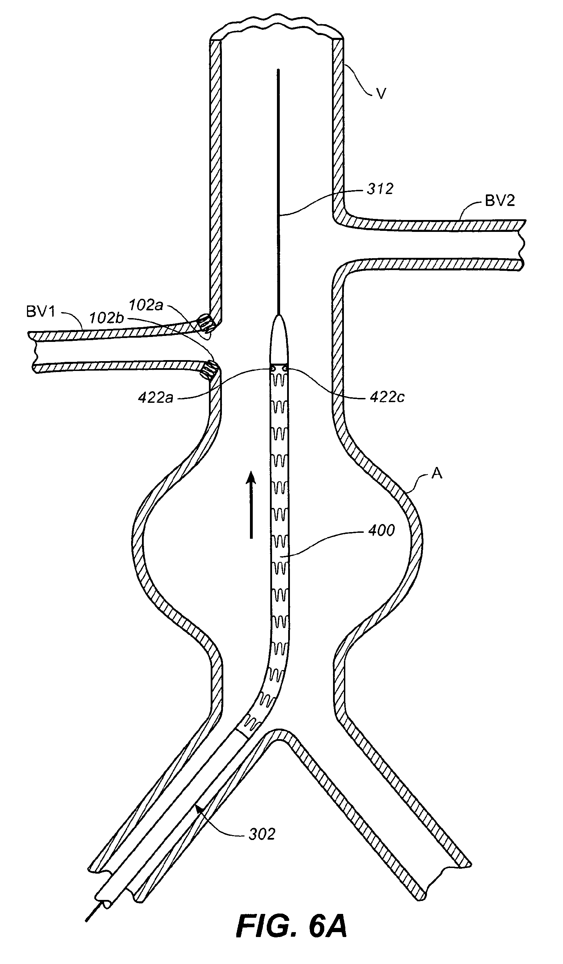

Referring to FIG. 6A, the operator advances guidewire 312 through the femoral artery up the aorta to the position shown and tracks catheter 302 over guidewire 312 toward aneurysm A and branch vessels BV1 and BV2, which branch from vessel V.

The relative positions of implanted markers 102a, 102b, and 102c and stent-graft markers 422a, 422b, and 422c are monitored based on the marker signals received and measured by sensors 626 in sensor array 616 (FIG. 10A). The sensors are coupled to signal processor 628 (FIG. 10A), which utilizes the marker signals 622 from sensors 626 to calculate the location of each marker 102a, 102b, 102c, 422a, 422b, and 422c (each of which structurally correspond to marker 614 shown in FIG. 10C) in three-dimensional space relative to a known frame of reference-the sensor array 616. After processing the signals the position data can be displayed on a monitor (not shown), which can be coupled to signal processing device 628 (FIG. 10A). The monitor can display virtual images of the markers and/or the structures to which they are attached or it can display numerical values indicative of the relative positions of the markers, or both. Regarding displaying structural images of the stent-graft, the stent-graft dimensions and relative positions of the markers attached thereto can be input into the processor prior to the procedure

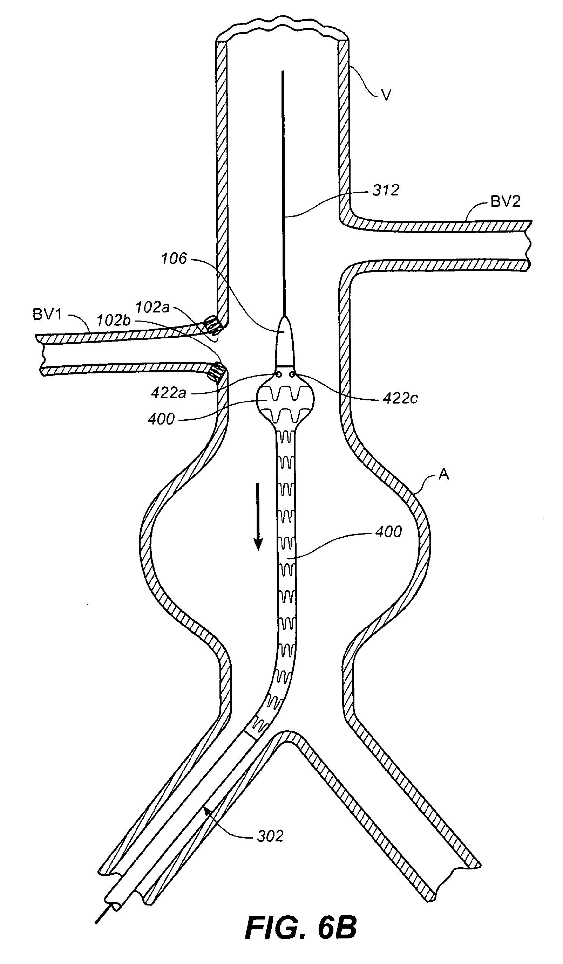

Referring to FIG. 6B, once the stent-graft markers are in the desired position relative to the implanted markers, the operator holds guidewire tube 310 and pusher disk 310 stationary, and retracts or pulls back catheter sheath 303. As the sheath is pulled back, the relative positions of the prosthesis markers relative to the implant markers are monitored to determine if movement beyond a threshold value occurs during deployment. Alternatively, the processor can display a warning when the prosthesis markers move more than a threshold value (e.g., more than 2mm) in the direction of blood flow or in a direction generally parallel to the central axis of the proximal end portion of the stent-graft. In a further alternative, the distance between the base of the ostia and the stent-graft markers, measured in the direction of blood flow or along the aortic wall in a distal direction (away form the heart), is displayed to provide a quantitative value of stent-graft movement in that direction. A non-symmetric marker array pattern will provide a precise incremental image of any rotation or other translation of the prosthesis. In contrast to a symmetrical array whose image would repeat and might be indistinguishable if it were rotated, by exactly one incremental mark. In yet a further alternative, the annular proximal end of the stent-graft can be monitored based on stent-graft dimensional data and the position of the markers relative to the proximal end of the stent-graft, which can be readily input into the processor.

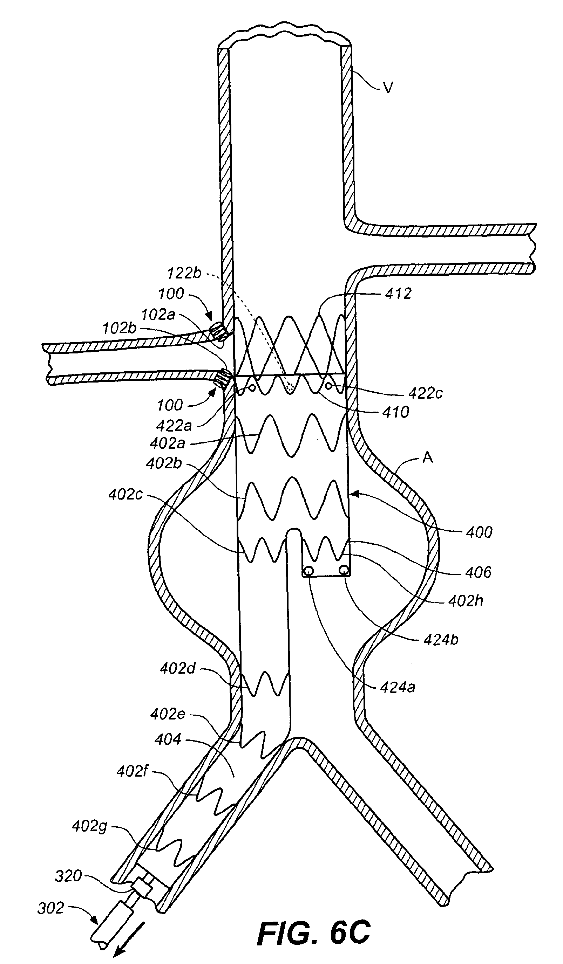

If the position of the stent-graft proximal portion changes more than a desired amount, the operator repositions the stent-graft. This can involve moving the sheath over the expanded portion of the stent-graft to allow the stent-graft to be more readily repositioned or simply repositioning the stent-graft before full deployment. The stent-graft is then deployed again while monitoring movement during deployment. The fully deployed stent-graft illustrating ipsilateral leg 404, contralateral stump 406, stent elements 402a-h, and support wires 410 and 412 is shown in FIG. 6C. As depicted in FIG. 6C, stent-graft contralateral stump 406 optionally can include one or more leadless electromagnetic markers such as markers 424a and 424b to assist with contralateral gate cannulation, which otherwise is performed using fluoroscopic techniques. Markers 424a and 424b provide targets that can be readily seen with the imaging system described herein such that the location of the contralateral gate in real-time can readily be displayed and a guidewire more readily aligned with and advanced through the gate as will be described in more detail below.

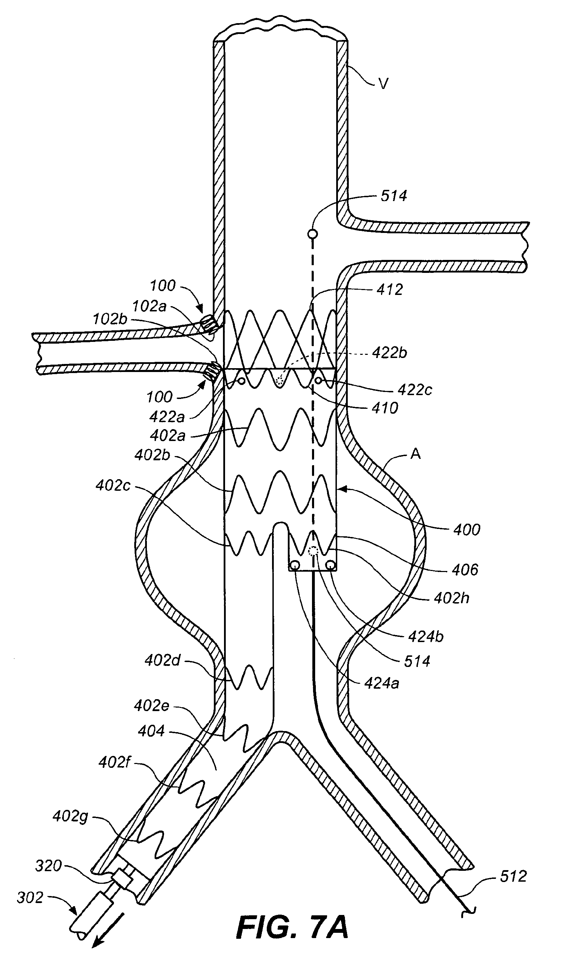

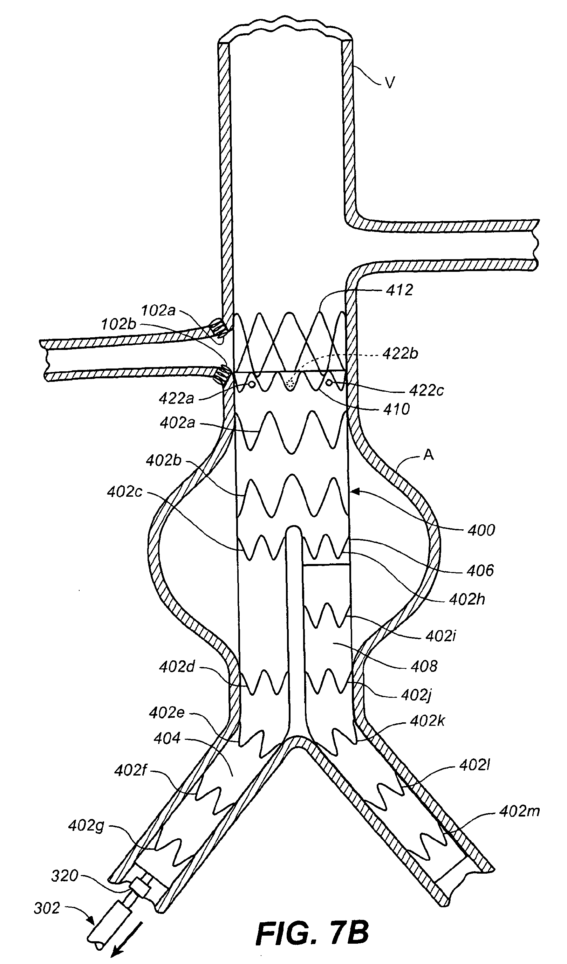

Referring to FIGS. 7A and 7B, a method of cannulating a contralateral stump of a bifurcated stent-graft using lead less electromagnetic markers will be described. A guidewire 512 having a leadless electromagnetic marker attached thereto at its distal end or adjacent to the distal end thereof with biocompatible adhesive or other suitable means is endovascularly advanced from the other femoral artery toward the contralateral stump 406. The relative positions of contralateral gate markers 424a,b and guidewire marker 514 are monitored based on the signals received from these markers (each of which structurally correspond to marker 614 in FIG. 10C) and measured by sensors 626 in sensor array 616 (FIG. 10A). The sensors are coupled to signal processor 628, which utilizes the marker signals 622 from sensors 626 to calculate the location of each marker in three-dimensional space relative to a known frame of reference-the sensor array 616. After processing the signals the position data can be displayed on a monitor (not shown), which can be coupled to signal processing device 628 (FIG. 10A). The monitor can display virtual images of the markers and/or the structures to which they are attached or it can display numerical values indicative of the relative positions of the markers, or both. Regarding displaying structural images of the contralateral gate and/or the guidewire, contralateral gate and/or guidewire dimensions and positions of the markers attached thereto can be input into the processor prior to the procedure.

Guidewire marker 514 is tracked toward markers 422a,b and passed through the contralateral gate. After passing through the gate, the guidewire is further advanced to enable guiding contralateral leg 408 thereover and into the contralateral stump 406. Contralateral stent-graft leg 408 is loaded in a delivery catheter which can be similar or the same as delivery catheter 302, and the delivery catheter tracked over guidewire 512. Once the contralateral stent-graft is in the desired position, it is deployed and allowed to expand in contralateral stump 406 as shown in FIG. 7B with its stent elements shown with reference characters 402i-m.

In placing the contralateral stent-graft at the desired position before deployment, conventional fluoroscopic techniques or leadless markers in accordance with a further embodiment of the invention can be used. In the latter case, the proximal end of the contralateral leg 406 can have leadless markers attached thereto and arranged as markers 422a-c are arranged about the proximal end portion of stent-graft 400 (e.g., equidistantly spaced and along the proximal end of the prosthesis). The position of the contralateral leg markers relative to markers 224a,b can be monitored as described above, and contralateral leg 408 held stationary when its desired position is achieved. According to another embodiment, a virtual model of the contralateral leg markers and contralateral stump markers when in the desired coupled relationship can be input into the processor prior to the procedure and the real-time position data of the markers using system 600 overlaid (using an image overlay) on that model to align the contralateral leg.

Although two markers 424a and 422b are shown, one or more markers can be used. In the case of a plurality of markers, they can be equidistantly spaced from one another. Further, any of the prosthesis markers described herein can be provided on the interior surface of the prosthesis or the exterior surface of the prosthesis.

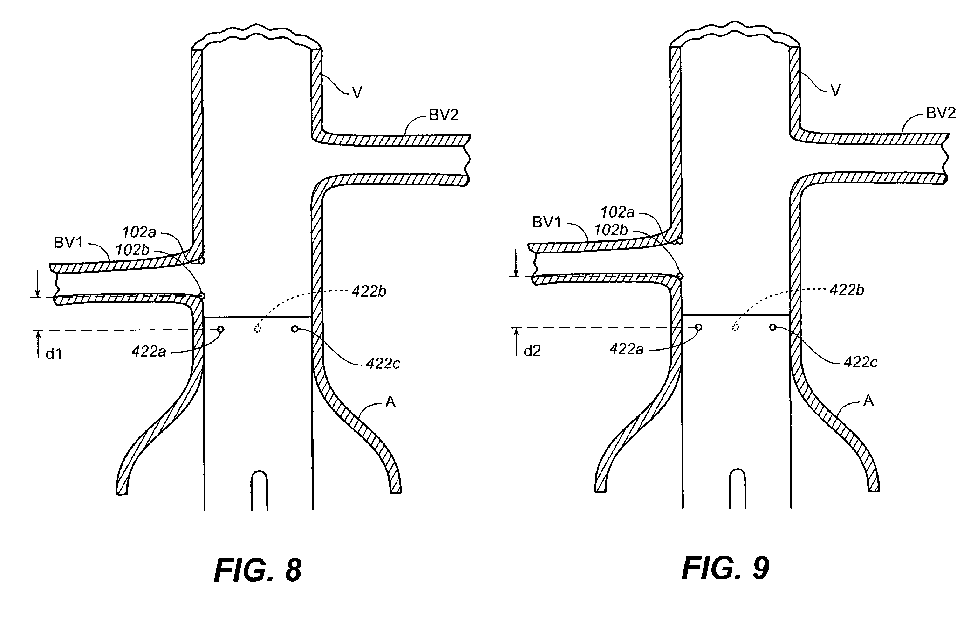

Referring to FIGS. 8 and 9, another embodiment of the invention will be described. In this embodiment, the location and relative position of the implanted and prosthesis proximal markers is recorded immediately after deployment as a first data set so that it can be compared to a second data set of the location and relative positions of the implanted and prosthesis proximal markers acquired sometime in the future. If there is variation between the two sets of data, then stent-graft migration or vessel elongation is indicated and more invasive follow up should be provided - any noted change in the distance between markers 102b and 422a would indicate a need for further follow up. Referring to FIG. 9, distance d1 between the plane defined by markers 422a-c and one of markers 102a-c (e.g., marker 102a, which is closest to the base of the branch vessel ostia) is measured and recorded using position localization system 600 after deployment. During long term post operative follow-up, the same dimension is measured using the same markers and position localization system 600. In the illustrative example in FIG. 10, this dimension is shown as d2, which when compared to d1 shows an increase in the dimension and indicates stent-graft migration or vessel elongation.

FIGS. 10A-C illustrate a system and components for generating an excitation signal for activating a resonating marker assembly and locating the marker in three-dimensional space which can be used in systems for performing methods in accordance with aspects of the present invention.

FIG. 10A is a schematic view of a system 600 for energizing and locating one or more leadless resonating marker assemblies 614 in three-dimensional space relative to a sensor array 616 where one marker assembly 614 is shown in this example. System 600 includes a source generator 618 that generates a selected magnetic excitation field or excitation signal 620 that energizes each marker assembly 614. Each energized marker assembly 614 generates a measurable marker signal 622 that can be sufficiently measured in the presence of both the excitation source signal and environmental noise sources. The marker assemblies 614 can be positioned in or on a selected object in a known orientation relative to each other. The marker signals 622 are measured by a plurality of sensors 626 in the sensor array 616 (see FIG. 10B). The sensors 626 are coupled to a signal processor 628 that utilizes the measurement of the marker signals 622 from the sensors 626 to calculate the location of each marker assembly 614 in three-dimensional space relative to a known frame of reference, such as the sensor array 616.

Source generator 618 is configured to generate the excitation signal 620 so that one or more marker assemblies 614 are sufficiently energized to generate the marker signals 622. The source generator 618 can be switched off after the marker assemblies are energized. Once the source generator 618 is switched off, the excitation signal 620 terminates and is not measurable. Accordingly, sensors 626 in sensor array 616 will receive only marker signals 622 without any interference or magnetic field distortion induced by the excitation signal 620. Termination of the excitation signal 620 occurs before a measurement phase in which marker signals 622 are measured. Such termination of the excitation signal before the measurement phase when the energized marker assemblies 614 are generating the marker signals 622 allows for a sensor array 616 of increased sensitivity that can provide data of a high signal-to-noise ratio to the signal processor 628 for extremely accurate determination of the three-dimensional location of the marker assemblies 614 relative to the sensor array or other frame of reference.

The miniature marker assemblies 614 in the system 600 are inert, activatable assemblies that can be excited to generate a signal at a resonant frequency measurable by the sensor array 616 remote from the target on which they are placed. The miniature marker assemblies 614 have, as one example, a diameter of approximately 2 mm and a length of approximately 5 mm, although other marker assemblies can have different dimensions. An example of such a marker detection systems are described in detail in U.S. Patent Publication No. 20020193685 entitled Guided Radiation Therapy System, filed Jun. 8, 2001 and published on December 19, 2002, and U.S. Patent No. 6,822,570 to Dimmer et al., entitled System For Spacially Adjustable Excitation Of Leadless Miniature Marker, all of the disclosures of which are incorporated herein in their entirety by reference thereto.

Referring to FIG. 10C, the illustrated marker assembly 614 includes a coil 630 wound around a ferromagnetic core 632 to form an inductor (L). The inductor (L) is connected to a capacitor 634, so as to form a signal element 636. Accordingly, the signal element 636 is an inductor (L) capacitor (C) resonant circuit. The signal element 636 can be enclosed and sealed in an encapsulation member 638 made of plastic, glass, or other inert material. The illustrated marker assembly 614 is a fully contained and inert unit that can be used, as an example, in medical procedures in which the marker assembly is secured on and/or implanted in a patient's body as described in U.S. Patent No. 6,822,570 *(supra).*

The marker assembly 614 is energized, and thus activated, by the magnetic excitation field or excitation signal 620 generated by the source generator 618 such that the marker's signal element 636 generates the measurable marker signal 622. The strength of the measurable marker signal 622 is high relative to environmental background noise at the marker resonant frequency, thereby allowing the marker assembly 614 to be precisely located in three-dimensional space relative to sensor array 616.

The source generator 618 can be adjustable to generate a magnetic field 620 having a waveform that contains energy at selected frequencies that substantially match the resonant frequency of the specifically tuned marker assembly 614. When the marker assembly 614 is excited by the magnetic field 620, the signal element 636 generates the response marker signal 622 containing frequency components centered at the marker's resonant frequency. After the marker assembly 614 is energized for a selected time period, the source generator 618 is switched to the "off" position so the pulsed excitation signal 620 is terminated and provided no measurable interference with the marker signal 622 as received by the sensor array 616.

The marker assembly 614 is constructed to provide an appropriately strong and distinct signal by optimizing marker characteristics and by accurately tuning the marker assembly to a predetermined frequency. Accordingly, multiple uniquely tuned, energized marker assemblies 614 may be reliably and uniquely measured by the sensor array 616. The unique marker assemblies 614 at unique resonant frequencies may be excited and measured simultaneously or during unique time periods. The signal from the tuned miniature marker assembly 614 is significantly above environmental signal noise and sufficiently strong to allow the signal processor 628 (FIG 10A) to determine the marker assembly's identity, precise location, and orientation in three dimensional space relative to the sensor array 616 or other selected reference frame.

A system corresponding to system 600 is described in U.S. Patent No. 6,822,570 to Dimmer et al., entitled System For Spacially Adjustable Excitation Of Leadless Miniature Marker and which was filed August 7, 2002, the entire disclosure of which is hereby incorporated herein in its entirety by reference thereto. According to U.S. Patent No. 6,822,570, the system can be used in many different applications in which the miniature marker's precise three-dimensional location within an accuracy of approximately 1 mm can be uniquely identified within a relatively large navigational or excitation volume, such as a volume of 12 cm x 12cm x 12cm or greater. One such application is the use of the system to accurately track the position of targets (e.g., tissue) within the human body. In this application, the leadless marker assemblies are implanted at or near the target so the marker assemblies move with the target as a unit and provide positional references of the target relative to a reference frame outside of the body. U.S. Patent No. 6,822,570 further notes that such a system could also track relative positions of therapeutic devices (i.e., surgical tools, tissue, ablation devices, radiation delivery devices, or other medical devices) relative to the same fixed reference frame by positioning additional leadless marker assemblies on these devices at known locations or by positioning these devices relative to the reference frame. The size of the leadless markers used on therapeutic devices may be increased to allow for greater marker signal levels and a corresponding increase in navigational volume for these devices.

Other examples of leadless markers and/or devices for generating magnetic excitation fields and sensing the target signal are disclosed in U.S. Patent No. 6,889,833 to Seiler et al. and entitled Packaged Systems For Implanting Markers In A Patient And Methods For Manufacturing And Using Such Systems, U.S. Patent No. 6,812,842 to Dimmer and entitled Systems For Excitation Of Leadless Miniature Marker, U.S. Patent No. 6,838,990 to Dimmer and entitled Systems For Excitation Of Leadless Miniature Marker, U.S. Patent No. 6,977,504 to Wright et al. and entitled Receiver Used In Marker Localization Sensing System Using Coherent Detection, U.S. Patent No. 7,026,927 to Wright et al. and entitled Receiver Used In Marker Localization Sensing System And Having Dithering In Excitation, and U.S. Patent No. 6,363,940 to Krag and entitled System and Method For Bracketing And Removing Tissue all the disclosures of which are hereby incorporated herein in their entirety by reference thereto.

Another example of a suitable non-ionizing localization approach that accommodates wireless markers is the Calypso® 4D Localization System, which is a target localization platform based on detection of AC electromagnetic markers, called Beacon® transponders, which are implantable devices. These localization systems and markers have been developed by Calypso® Medical Technologies (Seattle, Washington).

In one example of a method of implanting an electromagnetic marker in a vessel of a patient, the method includes positioning an electromagnetic marker in a vessel of a patient; and fastening the electromagnetic marker to the vessel. Typically, a fastener is used to secure the electromagnetic marker to the vessel. In a typical example, the electromagnetic marker is secured to a branch artery, wherein the electromagnetic marker may be secured to the region of the branch artery where the branch artery branches from another artery.

In another example, a method of real-time imaging a portion of a vessel in a patient includes securing one or more leadless electromagnetic markers to a vessel of a patient, and generating electromagnetic fields about the one or more markers to detect the position of the one or more markers and create a virtual image of a portion of the vessel. Typically, the one or more markers are positioned sufficiently close to a branch vessel that branches form the vessel in which the marker is disposed to create a real-time virtual image of a portion of the branch ostia at the juncture of the vessel and branch vessel.

In still another example, a method of prosthesis deployment includes securing a first electromagnetic marker to a vessel of a patient; and tracking the first electromagnetic marker that is secured to the vessel with a second electromagnetic marker secured to a tubular prosthesis while endovascularly advancing the tubular prosthesis toward the first marker; and deploying the prosthesis when the relative position of the first and second markers is in a desired state. Typically, the prosthesis is deployed in a first vessel and the first marker is secured to a vessel that branches from the first vessel. Furthermore, the second vessel typically branches from the aorta. Typically, at a first time an electromagnetic field is used to detect the position of the markers after prosthesis deployment and at a second time an electromagnetic field is used to detect the position of the markers, and the positions are compared. In one example, the markers are leadless markers.

In another example, a method of cannulating a first tubular prosthesis with a second tubular prosthesis in vivo includes endovascularly positioning a first tubular prosthesis having an open end and an electromagnetic marker adjacent to said open end in a vessel; tracking the first electromagnetic marker that is secured to first the tubular prosthesis with a second electromagnetic marker secured to a guide while endovascularly advancing the guide toward the first marker and into the open end of the first tubular prosthesis; and guiding a second tubular prosthesis into the open end of the first tubular prosthesis with the guide. Typically, the guide is a guidewire and the second tubular prosthesis is tracked over the guidewire.

Any feature described in any one embodiment described herein can be combined with any other feature of any of the other embodiments whether preferred or not.

Variations and modifications of the devices and methods disclosed herein will be readily apparent to persons skilled in the art.

## Claims

1. A marker implant apparatus (100) comprising:
an electromagnetic marker (102) adapted for implantation in a human patient; and
a fastener (104) secured to said electromagnetic marker (102) and adapted to engage tissue, said fastener (104) having a helical configuration and a piercing end (104a) configured to pierce tissue.

2. A bifurcated tubular prosthesis (400) comprising:
a bifurcated tubular member having a proximal end and first and second tubular leg portions (404, 406) that extend in a direction distal to said proximal end, each tubular leg portion (404, 406) having an open end;
an electromagnetic marker (424a, 424b) secured to one of the tubular leg portions (406) in the vicinity of the open end of that portion.

3. The prosthesis of claim 2 wherein said electromagnetic marker (424a, 424b) is a leadless electromagnetic marker according to claim 1.

4. The prosthesis of claim 2 or 3 further including an electromagnetic marker (422a, 422b, 422c) secured to said to said prosthesis (400) in the vicinity of said proximal end.

5. The prosthesis of claim any of claims 2 to 4 wherein said prosthesis (400) is a bifurcated stent-graft.

6. A prosthesis delivery apparatus comprising:
a guidewire (512) adapted for endovascular delivery in a patient; and
a leadless electromagnetic marker (514) secured to said guidewire (512).

7. The apparatus of claim 6 wherein said electromagnetic marker (514) comprises a leadless coil.
